# EUROPEAN PATENT SPECIFICATION

(11) **EP 1 646 359 B1**
(45) Date of publication and mention of the grant of the patent: **25.04.2007**
(21) Application number: 04743903.9
(22) Date of filing: 05.07.2004
(51) Int. Cl.: A61Q 7/00, A61K 8/44, A61K 8/42, A61K 8/49, A61K 8/58, A61K 8/67

(54) **PREPARATION FOR TOPICAL USE WITH THE FUNCTION OF COMBATING HAIR LOSS**
ZUBEREITUNG ZUR ÄUSSERLICHEN ANWENDUNG ZUR BEKÄMPFUNG VON HAARAUSFALL
COMPOSITION A USAGE TOPIQUE SERVANT A LA LUTTE CONTRE LA CHUTE DES CHEVEUX

(30) Priority: 18.07.2003 CH 125803
(43) Date of publication of application: 19.04.2006
(73) Proprietor: Gecomwert Anstalt, 9490 Vaduz (LI)
(72) Inventor: MONTANARI, Daniela, I-35020 Albignasego-Padova (IT); GUGLIELMO, Manuela, I-30030 Vigonovo (IT)
(74) Representative: Fiammenghi-Domenighetti, Delfina
(86) International application number: PCT/IB2004/002241
(87) International publication number: WO 2005/007125

(56) References cited:
- EP-A- 1 205 184
- WO-A-97/07772
- FR-A- 2 740 331
- US-A- 2 718 028
- US-A- 5 157 036

## Description

This invention relates to the field of preparations for topical use used to combat and/or delay hair loss.
Hair loss represents detachment of the hair from the follicle and is a physiological phenomenon which forms part of the normal cyclical reproduction of hair.
On average physiological hair loss does not exceed 50-80 hairs per day. If the number of hairs lost is greater than this average then this constitutes anomalous hair loss.
Hair loss is a phenomenon causing extreme anxiety, for which remedies are sought through the use of products to prevent it.
This is the background to this invention; it relates in fact to a preparation for topical use, for cosmetic use, which is capable of combating hair loss through of its action targeted on the hair follicle, that is the structure which contains the root of the hair. The original feature of the preparation comprises its method of action which: 1) helps to promote the elasticisation of the follicle walls, 2) increases the anchoring of the hair root in the follicle, to hold it in place and prevent detachment.

In hair distinction is made between the shaft of the hair, or the free part, which projects from the external surface of the skin, and the root, which is implanted in the skin and held in the follicle. The deepest part of the root is called the bulb of the hair.
The hair therefore grows within an invagination in the epidermis of the skin, or the follicle, which therefore comprises an epidermal part and an external part which is continuous with the dermis. The hair follicle wall is therefore formed externally of a layer of connective tissue and internally of an epithelial layer.
From the outside inwards the follicle during anagen therefore comprises the following structures: connective tissue sheath, outer epithelial sheath, inner epithelial sheath.
The connective tissue sheath surrounds the follicle throughout its entire length continuing below into the connective tissue of the dermal papilla. Damage to the sheath may prevent a follicle engaging in normal anagen.

The connective tissue sheath is formed by a thickening of the connective tissue in which it is possible to distinguish, depending upon the orientation, collagen fibres and elastic fibres, an outer layer, an inner layer and a vitreous layer in contact with the cells of the follicle's epidermis.
The outer epithelial sheath continues the skin's epidermis and extends from the mouth of the follicle, that is from the point where the hair emerges, to the bulb, where it continues within the matrix. In the upper portion it has a structure similar to that of the epidermis; further down it is only formed by the basal and spinous layers. The follicle epidermis with the spinous layer is therefore in contact with the outermost layer of the sheath of the root (Henle's layer).
The inner epithelial sheath surrounds and covers the hair root as far as the mouth of the follicle, where it disintegrates and is removed. From the outside inwards it comprises three layers: Henle's layer (in contact with the epidermis of the follicles), Huxley's layer and the cuticle which adheres closely to the cuticle of the hair. The inner epithelial sheath can be regarded as being similar to the granular and lucid layers of the epidermis. Given the structural complexity of follicles it is obvious that satisfactory functioning of the biological components which are directly or indirectly involved in the follicle's structure is fundamental in order to hold the root within the follicle and to allow the hair to experience a correct life cycle, that of growth, involution and rest prior to falling out.

Transglutaminases are enzymes which are found in many cell compartments. The intermolecular cross-linking catalysed by transglutaminases is of extreme importance in the keratinisation of the epidermis and hairs.
Epidermal transglutaminases are located in the granular layer, where they have an important part to play; they stabilise the bond between lysine and glutamine residues in order to form bridges between different structural proteins. This protein-protein link is necessary to form the corneocytes' enclosure during the process of keratinisation. The glutamyl-lysine links represent an important marker for normal differentiation of the epidermis. In fact many of the characteristics attributed to these tissues, such as strength, stability and impermeability, are due in part to the presence of transglutaminases.
Follicular transglutaminases are located in the inner sheath of the root. These enzymes help through forming links between structural proteins, and consequent cross-linking, increasing the strength of the hair, especially at the base, where it is attached to the scalp. Disturbances in the formation of these crosslinks may result in hairs having diminished cohesion or tensile strength.

Because qualitative and quantitative damage to collagen and elastic fibres (fundamental components of the connective tissue) due to causes of various natures has an effect on the elasticity of the tissues, it is important to maintain the nutrition of the epidermal and connective tissue structures present in the sheaths forming the follicle and the tissues receiving and surrounding the follicle in the scalp. Cross-linking of the protein structures which helps to secure the root in the follicle through the formation of glutamyl-lysine bonds during the process of keratinisation which takes place in the sheaths is also important.
Disturbance or damage to these configurations may bring about major structural changes and diminished cohesion between the root and the follicle.

The preparation to which the invention relates is designed to slow down hair loss through the synergistic action of its functional components.
From a formulative point of view the invention makes use of all the known loss-preventing potential of a vasodilator, benzyl nicotinate, (which is fundamental to action to combat hair loss in that by locally increasing the blood flow it provides an adequate input of nutrient substances and oxygen to the hair) included in a water-alcohol vehicle enriched with pantenol and menthol.
However the original feature of the invention lies in its method of action and above all in the target of the action to combat hair loss, which is specifically the hair follicle. This action takes the following forms:
- it encourages elasticisation of the follicle walls in order to prevent them from hardening, thanks to the presence of two amino acids, hydroxyproline and aspartic acid, which maintain the function of the connective tissue structure surrounding and supporting the follicle,
- it helps to anchor the hair root in the follicle by holding it in its seat, delaying detachment and therefore loss thanks to the presence of an enzyme activator which acts on the transglutaminases.

It is known that skin damage is the result of exogenous and endogenous factors which are specific to each individual. Some harmful exogenous factors are UV rays, free radicals, ageing.

Cell activity as a whole decreases, causing qualitative and quantitative damage to the collagen and elastic fibres. Tissue regeneration decreases and hardening of the tissues occurs through damage to the cellular components making up the connective tissue which prevents sclerosis. All this is reflected in a diminution of the elasticity of the skin.
The two amino acids hydroxyproline and aspartic acid, included in a silanol, monomethylsilanol-hydroxyproline aspartate (silanols are organic derivatives of silicon, very rich in hydroxyl groups and synthesised in the presence of different radicals which give them stability and specificity), have a cytostimulant effect. They in fact encourage the process of cell division in the fibroblasts. The fibroblasts present in the dermis synthesise collagen, elastin and components of the extracellular matrix; an increase in their number may result in greater synthesis of these molecules.
It can therefore be said that hydroxyproline and aspartic acid when complexed in a silanol can contribute to improving and normalising the connective tissue structures restoring elasticity to skin tissues.

The inventors have considered it important to apply this action to the scalp by selecting monomethyl silanol-hydroxyproline aspartate as one of the active ingredients of the preparation according to the invention. As previously described, hair follicles are derived from an invagination in the skin and comprise an epidermal part and a connective tissue part in continuity with the dermis of the skin itself. This is the seat of action of the two amino acids, hydroxyproline and aspartic acid, which form part of the complex with the silanol.
Its purpose is to limit hardening of the tissues encouraging elasticisation of the follicle walls. In this way follicles are helped to maintain optimum physiological conditions for holding the hairs which they contain in their seats.
The cell-stimulating action of the complex of amino acids with silanol has been tested on human fibroblast cultures. The increase in cell growth was 46% when that active ingredient was present in the culture medium.
It is also pointed out that silicon, as a component of the complex described above, is an essential element in the mucopolysaccharide-protein complexes of connective tissue. Acting as a cross-linking agent, silicon can contribute to the structural integrity of the connective tissue which comes in contact with the hair bulb.

The inventors then identified octyl butyrate as the socalled enzyme activator, that is the molecule capable of stimulating the activity of transglutaminases in the cells of the scalp in order to help increase the anchoring of hairs at their attachment sites.
The choice of octyl butyrate came from the observation that some molecules are capable of inducing transglutaminase synthesis; this led the researchers to investigate the case of sodium butyrate. This substance is capable of stimulating the synthesis of transglutaminases in keratinocytes and formation of the corneified enclosure of the corneocytes (keratinocytes in the final stage of differentiation).
Being a substance which cannot be used in cosmetics because of its bad smell, its effects have been reproduced by an ester of butyric acid, octyl butyrate, an ester of octanol and butyric acid, which is then hydrolysed by skin enzymes such as esterases.

The role of octyl butyrate, the enzyme activator in the invention in question, is that of stimulating the activity of transglutaminases in the cells of the scalp in order to help increase the anchoring of hairs to their sites of attachment.

Peptides rich in glutamine, derived from cereals, enrich the preparation to which the invention relates and contribute synergistically to the activity of the octyl butyrate. They in fact represent an excellent substrate for transglutaminases.
An additional comment concerning the above glutamine peptides: the amino acid glutamine is regarded as being the "fuel" of cell emergence, both when glucose is deficient and when the cells are in a stage of strong growth and multiplication. This is the situation of the hair follicle cells during the anagen phase. The glutamine peptides therefore support the octyl butyrate in the preparation in question by acting as an energy supplement for the cells which are undergoing strong multiplication.

With the specific object of evaluating the hair loss-combating activities of this specific active ingredient a self-assessment trial was performed on 20 volunteers for three months against placebo, which revealed the following:
- in the case of the treated group:
   before treatment most of the volunteers had visible alopecia. The amount of hair loss was great in 22%, average in 67% and small in 11%.
   After 3 months treatment with the lotion containing enzyme activator 67% of the volunteers observed a decrease in hair loss.
- in the case of the placebo group:
   77% of the volunteers had visible alopecia. The amount of hair loss was average for 66% and small for 33%.
   After 3 months use of the placebo lotion the amount of hair loss appeared to be unchanged. No visible improvements were observed.

The hair loss-combating effectiveness of the preparation in its entire formulation was tested through a clinical trial conducted by a specialised institute.
20 volunteers of the male and female sex having telogen effluvium problems were selected for the purpose and used the preparation once a day on alternate days for 60 consecutive days.
At the start of the test, after 1 month's treatment, and after 60 days of use, the degree of resistance to traction was clinically evaluated by the Pull test and hairs lost after washing of the scalp under controlled conditions were counted (Wash test).
An objective examination of the condition of the scalp, a subjective evaluation of effectiveness by the volunteers and a psychological evaluation relating to satisfaction were also performed.
Analysis of the results revealed:
- a statistically significant increase in resistance to traction both after 30 days and after 60 days application of the preparation
- a statistically significant decrease in the number of hairs lost during washing both after 30 days and after 60 days of application of the preparation
- from the subjective point of view the volunteers observed a decrease in the fall-out of hairs as well as a decrease in the level of seborrhoea.

The subject matter of this invention therefore comprises a preparation as described in appended claim 1.
A description of a preferred embodiment of the preparation according to the invention in which all the components and active ingredients described previously are present will now be provided.
The composition of this embodiment comprises:
- between 0.001 and 0.2% by weight of octyl butyrate,
- between 0.001 and 0.2% by weight of glutamine peptides,
- between 0.001 and 0.5% by weight of monomethylsilanol-hydroxyproline aspartate,
- between 0.05% and 0.15% by weight of benzyl nicotinate,
- between 0.05% and 0.5% by weight of pantenol.

A liquid vehicle, for example ethyl alcohol and water, one or more perfumed essences, such as menthol and the like, and preservatives are added to this composition.
The preparation may find specific indication for different stages of severity of hair loss using increasing percentages of the active ingredients (octyl butyrate, glutamine peptides, monomethylsilanol-hydroxyproline aspartate, benzyl nicotinate) in proportion to the increased intensity of hair loss.

## Claims

1. Preparation for topical use containing benzyl nicotinate and having the function of combating and/or delaying hair loss, **characterised in that** its composition comprises:
- two amino acids, hydroxyproline and aspartic acid, complexed with a silanol,
- an enzyme activator, comprising octyl butyrate.

2. Preparation according to claim 1, the composition of which also includes glutamine peptides.

3. Preparation according to one of the preceding claims, the composition in which also comprises pantenol.

4. Preparation according to claim 3, **characterised in that** its composition comprises:
a) octyl butyrate in an amount between 0.001 and 0.2% by weight,
b) glutamine peptides in an amount between 0.001 and 0.2% by weight,
c) monomethylsilanol-hydroxyproline aspartate in an amount between 0.001 and 0.5% by weight,
d) benzyl nicotinate in an amount between 0.05 and 0.15% by weight,
e) pantenol in an amount between 0.05 and 0.5% by weight.

5. Preparation according to claim 4, in which a liquid vehicle, one or more perfumed substances and preservatives are added.

6. Preparation according to claim 5, to which ethyl alcohol, water, menthol and preservatives are added.

## Patentansprüche

1. Präparat zur topischen Verwendung, enthaltend Benzylnikotinat, und mit der Funktion, Haarausfall zu bekämpfen und/oder zu verzögern, **dadurch gekennzeichnet, dass** seine Zusammensetzung umfasst:
zwei Aminosäuren, Hydroxyprolin und Asparaginsäure, in einem Komplex mit einem Silanol,
- einen Enzymaktivator, umfassend Octylbutyrat.

2. Präparat nach Anspruch 1, wobei die Zusammensetzung auch Glutamin-Peptide beinhaltet.

3. Präparat nach einem der vorhergehenden Ansprüche, wobei die Zusammensetzung auch Pantothenol umfasst.

4. Präparat nach Anspruch 3, **dadurch gekennzeichnet, dass** seine Zusammensetzung umfasst:
a) Octylbutyrat in einer Menge zwischen 0,001 und 0,2 Gew.-%,
b) Glutamin-Peptide in einer Menge zwischen 0,001 und 0,2 Gew.-%,
c) Monomethylsilanol-Hydroxyprolin-Aspartat in einer Menge zwischen 0,001 und 0,5 Gew.-%,
d) Benzylnikotinat in einer Menge zwischen 0,05 und 0,15 Gew.-%,
e) Pantothenol in einer Menge zwischen 0,05 und 0,5 Gew.-%.

5. Präparat nach Anspruch 4, dem ein flüssiges Vehikel, eine oder mehrer Duftsubstanzen und Konservierungsstoffe zugesetzt sind.

6. Präparat nach Anspruch 5, dem Ethylalkohol, Wasser, Menthol und Konservierungsstoffe zugesetzt sind.

## Revendications

1. Préparation pour usage topique contenant du nicotinate de benzyle et ayant pour fonction de combattre et/ou de retarder la chute des cheveux, **caractérisée en ce que** sa composition comprend :
- deux acides aminés, l'hydroxyproline et l'acide aspartique, complexés avec un silanol,
- un activateur d'enzyme comprenant le butyrate d'octyle.

2. Préparation selon la revendication 1, dont la composition comprend également des glutamine peptides.

3. Préparation selon l'une des revendications précédentes, dont la composition comprend également du panténol.

4. Préparation selon la revendication 3, **caractérisée en ce que** sa composition comprend :
a) du butyrate d'octyle dans une quantité comprise entre 0,001 et 0,2 % en poids,
b) des glutamine peptides dans une quantité comprise entre 0,001 et 0,2 % en poids,
c) du monométhylsilanol-aspartate d'hydroxyproline dans une quantité comprise entre 0,001 et 0,5 % en poids,
d) du nicotinate de benzyle dans une quantité comprise entre 0,05 et 0,15 % en poids,
e) du panténol dans une quantité comprise entre 0,05 et 0,5 % en poids.

5. Préparation selon la revendication 4, dans laquelle sont ajoutés un véhicule liquide, une ou plusieurs substances parfumées et des conservateurs.

6. Préparation selon la revendication 5, à laquelle sont ajoutés de l'alcool éthylique, de l'eau, du menthol et des conservateurs.
